Europäisches Patentamt

European Patent Office

Office européen des brevets

(19)

(11) Veröffentlichungsnummer: **0 225 483**
**B1**

(12) # EUROPÄISCHE PATENTSCHRIFT

(45) Veröffentlichungstag der Patentschrift:
**24.01.90**

(51) Int. Cl.⁴: **A61K 6/04, C22C 5/02**

(21) Anmeldenummer: **86115314.6**

(22) Anmeldetag: **05.11.86**

(54) **Dental-Goldlegierungen.**

(30) Priorität: **07.12.85 DE 3543285**

(43) Veröffentlichungstag der Anmeldung:
**16.06.87 Patentblatt 87/25**

(45) Bekanntmachung des Hinweises auf die Patenterteilung:
**24.01.90 Patentblatt 90/4**

(84) Benannte Vertragsstaaten:
**AT BE CH DE FR GB IT LI LU NL SE**

(56) Entgegenhaltungen:
**DE-A- 2 453 799**
**DE-A- 2 509 476**
**DE-A- 2 636 039**

(73) Patentinhaber: **Degussa Aktiengesellschaft,**
**Weissfrauenstrasse 9, D-6000 Frankfurt am Main 1(DE)**

(72) Erfinder: **Kropp, Rudolf, Dr., Hölderlinstrasse 2,**
**D-7530 Pforzheim(DE)**
Erfinder: **Schiwiora, Harry, Theodor-Heuss-Strasse 37,**
**D-7530 Pforzheim(DE)**

## Beschreibung

Die Erfindung betrifft Dental-Goldlegierungen, insbesondere zur Herstellung von abnehmbaren Brücken. Daneben können sie aber auch für andere Kronen- und Brückenarbeiten eingesetzt werden.

Unter abnehmbaren Brücken versteht man in der Zahntechnik Brücken mit zweiteiligen Kronen, von denen der Primärteil zylindrisch oder konusförmig gestaltet ist und auf den präparierten Zahnstumpf fest aufzementiert wird. Der Sekundärteil wird passend zum Primärteil innen zylindrisch oder konisch gestaltet, während die Außenflächen die Konturen des ursprünglichen Zahns nachgestalten. Dieser Sekundärteil ist Ankerteil einer Brücke, die durch diese Konstruktion herausnehmbar ist und durch die Friktion zwischen den Zylindern oder Koni von Primär- und Sekundärkronen festgehalten wird. Vielfach wird die Friktion auch noch durch individuell gefräste Geschiebe verbessert.

Zur Herstellung solcher Kronen und Brücken werden seit langem Gold-Platin-Legierungen mit einer Härte von mindestens l50 HV eingesetzt. Die erforderliche Härte wird vor allem durch den Zusatz von 7 - l2 % Kupfer erreicht, da der Platinzusatz dazu allein nicht ausreicht, wenn das Schmelzintervall nicht zu stark erhöht werden soll.

Im Munde eingesetzte herausnehmbare Brücken bilden stets zwischen Primär- und Sekundärteil einen Spalt aus. Dieser füllt sich mit Speichel, der an Luftsauerstoff verarmt. Hierdurch kommt es zur Ausbildung eines Belüftungselementes, das die Innenflächen anodisch und die Außenflächen kathodisch werden läßt. Diese Spannungsdifferenz läßt Kupfer aus der Legierung an den Innenflächen in Lösung gehen und sich am Spaltaustritt wieder metallisch abscheiden. Hierdurch kommt es zu unaesthetischen Verfärbungen an den Außenflächen der Kronen. Es bestand daher schon seit langem der Wunsch, für herausnehmbare Brücken kupferfreie Legierungen einsetzen zu können.

Kupferfreie Dentalgoldlegierungen sind in den DE-PSen 2l 39 33l, 24 53 799 und 25 09 476 beschrieben. Sie enthalten Unedelmetalle in Form von Indium, Zinn und Zink. Diesen Legierungen haftet jedoch der Nachteil an, daß die Unedelmetallzusätze zur Erreichung der erforderlichen Mindesthärte beim Vergießen schwer lösliche bis unlösliche Oxidschichten auf der Oberfläche bilden und an den Korngrenzen entlang in die Oberfläche eindringen (Indium, Zinn). Legierungen gemäß der DE-PS 25 09 476 enthalten einen hohen Anteil an Gold und Platin, was die Legierungen sehr teuer werden läßt. Darüber hinaus haben diese bekannten Legierungen den Nachteil, daß ihre Bruchdehnung im gegossenen und/oder ausgehärteten Zustand, d.h. ohne Tempern mit anschließendem Abschrecken, mit nur 3 - 5 % recht niedrig liegt, so daß es leicht zu Beschädigungen durch Sprödbrüche kommen kann. Diese Gefahr besteht vor allem dann, wenn die zervikalen Ränder der Sekundärkronen dünn modelliert sind und es beim Einsetzen der Brücke zu Verkantungen kommt.

Es war daher Aufgabe der vorliegenden Erfindung, Dental-Goldlegierungen zu entwickeln, insbesondere zur Herstellung von abnehmbaren Brücken, die zur Härtesteigerung nur solche Unedelmetalle enthalten, die keine Verfärbungen hervorrufen, keine säureunlöslichen Oxidschichten bilden und keine Oberflächenbeeinflussungen und Versprödungen durch Korngrenzenoxide bewirken. Außerdem sollte der Gold- und Platingehalt möglichst niedrig liegen und die Bruchdehnung der Legierungen einen möglichst hohen Wert aufweisen.

Diese Aufgabe wird erfindungsgemäß dadurch gelöst, daß sie aus 50 bis 6l % Gold, 20 bis 35 % Silber, l0 bis l5 % Palladium, 0 bis 2 % Platin, 0 bis 0,2 % Iridium und/oder Ruthenium und 3 bis 5 % Zink bestehen, wobei die Summe von Gold, Platin und Palladium 74 % nicht überschreiten darf.

Vorzugsweise bestehen die Dental-Goldlegierungen aus 55 bis 60 % Gold, 24 bis 28 % Silber, ll bis l4 % Palladium, 0,05 bis 0,l % Iridium und/oder Ruthenium und 4 bis 5 % Zink.

Die Legierungen können auf Kosten des Palladiumgehaltes bis zu 2% Platin enthalten. Die erfindungsgemäßen Legierungen bilden überraschenderweise im Gegensatz zu den bekannten kupferfreien Legierungen beim Vergießen keine säurelöslichen Oxide auf der Oberfläche, die die Qualität der Oberfläche zudem durch Aufrauhung und Eindringen in die Tiefe deutlich verschlechtern könnten. Die Legierungen sind nach dem Ausbetten aus der Gußmuffel blank, brauchen nicht mehr abgesäuert zu werden und erfordern keine mechanische Nachbearbeitung der exakt modellierten Innenflächen von Sekundärkronen, welche die Paßgenauigkeit auf dem Primärteil nachteilig zu beeinflussen pflegen. Im Gegensatz zu allen bisher bekannten kupferfreien Dentalgoldlegierungen haben die erfindungsgemäßen Legierungen überraschenderweise eine relativ hohe Bruchdehnung (siehe Tabelle) und damit eine gute Duktilität, wie sie bislang nur bie kupferhaltigen Legierungen erreicht werden konnte. Dadurch ist die Gefahr von Brüchen beim Ausbetten der Gußobjekte, beim Ausarbeiten und beim Ein- und Ausgliedern der fertigen Brücken deutlich vermindert. Die Bruchgefahr wird weiterhin vermindert durch die glatte Gußoberfläche, da entlang der Korngrenzen keine Oxide eindringen und damit keine Ansatzstellen für Beginn und Ausbreitung von Rissen schaffen können.

In der Tabelle sind einige Beispiele für erfindungsgemäße Legierungen aufgeführt, von denen die Legierungen 4 eine besondere hohe Härte bereits im gegossenen Zustand ohne zusätzliche Wärmebehandlung zeigt, während die Legierungen l, 3 und 5 besonders hohe Bruchdehnungen bei immer noch ausreichender Härte aufweisen. Die Bruchdehnungen der bekannten, kupferfreien Dental-Goldlegierungen liegen bei 3 bis 5,5 %.

Durch den Zusatz von Iridium und/oder Ruthenium läßt sich die Kornzahl auf über l000 Körner/mm² steigern.

Die erfindungsgemäßen Legierungen sind von einer äesthetisch angenehmen hellgelben Farbe. Sie sind außer für abnehmbare Brücken auch für die normale Kronen- und Brückentechnik ohne herausnehmbare Teile geeignet und bieten dort den Vorteil, daß Spalten zwischen Legierung und Kunststoffverblendung oder zwischen Legierung und Gingiva oder Oberflächenlunker und Porositäten ebenfalls keinen Anlaß zur Verfärbung durch Bildung von Belüftungselementen geben können.

Tabelle

| Legierung (Zusammensetzung %) | 1 | 2 | 3 | 4 | 5 | 6 | 7 |
|---|---|---|---|---|---|---|---|
| Au | 55,0 | 55,0 | 55,0 | 60,0 | 50,0 | 55,0 | 55,0 |
| Pt | — | — | — | — | 1,0 | — | 1,0 |
| Pd | 9,9 | 11,9 | 13,9 | 11,9 | 11,9 | 11,9 | 13,9 |
| Ir | 0,1 | 0,1 | 0,1 | 0,1 | 0,1 | 0,1 | — |
| Ru | — | — | — | — | — | — | 0,1 |
| Ag | 31,0 | 29,0 | 27,0 | 24,0 | 33,0 | 28,0 | 27,0 |
| Zn | 4,0 | 4,0 | 4,0 | 4,0 | 4,0 | 5,0 | 3,0 |
| Schmelzintervall (°C) | 1050-945 | 1060-975 | 1105-995 | 1095-975 | 1070-965 | 1045-935 | 1135-1035 |
| Härte HV Gußzustand | 160 | 175 | 185 | 200 | 160 | 185 | 175 |
| ·Bruchdehnung Gußzustand (%) | 12,2 | 9,0 | 12,2 | 8,5 | 12,9 | 9,0 | 10,9 |

## Patentansprüche

1. Dental-Goldlegierungen, insbesondere zur Herstellung von abnehmbaren Brücken,
dadurch gekennzeichnet,
daß sie aus 50 - 6l % Gold, 20 - 35 % Silber, l0 - l5 % Palladium, 0 - 2 % Platin, 0 - 0,2 % Iridium und/oder Ruthenium und 3 - 5 % Zink bestehen, wobei die Summe von Gold, Platin und Palladium 74 % nicht überschreiten darf.

2. Dental-Goldlegierungen nach Anspruch l,
dadurch gekennzeichnet,
daß sie aus 55 - 60 % Gold, 24 - 28 % Silber, ll - l4 % Palladium, 0,05 - 0,l % Iridium und/oder Ruthenium und 4 - 5 % Zink bestehen.

## Claims

1. Dental-gold alloys, especially for the manufacture of removable bridges, characterised in that they consist of 50–61% gold, 20–35% silver, 10–15% palladium, 0–2% platinum, 0–0.2% iridium and/or ruthenium and 3–5% zinc, where the gold, platinum and palladium together may not exceed 74%.

2. Dental-gold alloys according to claim 1, characterised in that they consist of 55–60% gold, 24–28% silver, 11–14% palladium, 0.05–0.1% iridium and/or ruthenium and 4–5% zinc.

## Revendications

1. Alliages d'or dentaires en particulier pour des bridges amovibles, caractérisées en ce qu'ils se composent de:
50 à 61% d'or
20 à 35% d'argent
10 à 15% de palladium
0 à 2% de platine
0 à 0,2% d'iridium et/ou de ruthénium et
3 à 5% de zinc
pour lesquels la somme de l'or, du platine, et du palladium ne doit pas dépasser 74%.

2. Alliages d'or dentaires selon la revendication 1, caractérisés en ce qu'ils se composent de 55 à 60% d'or, 24 à 28% d'argent, 11 à 14% de palladium, de 0,05 à 0,1% d'iridium et/ou de ruthénium de 4 à 5% de zinc.